# DEMANDE DE BREVET EUROPEEN

(11) **EP 4 394 047 A1**
(43) Date de publication de la demande: **03.07.2024**
(21) Numéro de dépôt: 23219362.3
(22) Date de dépôt: 21.12.2023
(51) Int. Cl.: C12Q 1/6844

(54) **MÉTHODE POUR CONTRÔLER LA QUALITÉ D'UNE AMPLIFICATION LAMP ET KIT UTILISÉ A CET EFFET**

(30) Priorité: 27.12.2022 FR 2214563
(71) Demandeur: Commissariat à l'énergie atomique et aux énergies alternatives, 75015 Paris (FR); Centre National de la Recherche Scientifique, 75016 Paris (FR); UNIVERSITÉ GRENOBLE ALPES, 38400 Saint-Martin-d'Heres (FR)
(72) Inventeur: AUBRET, Mathilde, 38054 GRENOBLE CEDEX 09 (FR); BOURDAT, Anne-Gaëlle, 38054 GRENOBLE CEDEX (FR); BUHOT, Arnaud, 38054 GRENOBLE CEDEX 09 (FR); CUBIZOLLES, Myriam-Laure, 38054 GRENOBLE CEDEX 09 (FR); ROUPIOZ, Yoann, 38570 THEYS (FR); BENCHETRIT, Hugo, 38054 GRENOBLE Cedex 09 (FR); SAVONNET, Maud, 38054 GRENOBLE CEDEX 09 (FR)
(74) Mandataire: Brevalex

(57) **Abrégé**

La présente invention concerne un procédé pour contrôler la qualité d'une amplification isotherme médiée par des boucles d'un mélange réactionnel contenant un contrôle interne et éventuellement au moins une séquence nucléotidique d'intérêt, ledit procédé comprenant : a) la co-amplification isotherme médiée par des boucles dudit contrôle interne et de ladite séquence nucléotidique d'intérêt éventuellement présente dans ledit mélange réactionnel et b) le contrôle de la qualité de l'amplification isotherme médiée par des boucles en détectant le produit de l'amplification dudit contrôle interne se présente sous forme d'un oligonucléotide de formule (I) et le mélange réactionnel contenant au plus trois amorces utiles pour l'amplification isotherme médiée par des boucles et la détection dudit contrôle interne. La présente invention concerne également un kit pour un tel contrôle qualité.

## Description

### DOMAINE TECHNIQUE

La présente invention concerne le domaine des outils et procédés de détection de molécules d'intérêt.

Plus particulièrement, la présente invention propose un oligonucléotide utile comme contrôle interne dans un procédé pour contrôler la qualité d'une amplification isotherme médiée par des boucles ou LAMP (pour « Loop-mediated isothermal AMPlification ») ou encore amplification LAMP. Cet oligonucléotide peut également être associé, dans un kit, aux deux ou trois amorces utiles pour son amplification et/ou la détection de son amplification lors de cette LAMP.

### ÉTAT DE LA TECHNIQUE ANTÉRIEURE

De nombreux travaux et études dans des domaines englobant notamment la recherche académique ; la recherche et les analyses, cliniques et diagnostiques, avec des essais réalisés sur des prélèvements de sang ou des biopsies cellulaires ou tissulaires; la surveillance environnementale ; la surveillance civile ; la sécurité alimentaire et notamment le contrôle qualité ; les analyses criminelles sur traces... impliquent la détection de biomarqueurs, de molécules d'intérêt ou de contaminants dans un échantillon et notamment dans un échantillon tel qu'un échantillon liquide.

Plusieurs techniques ont été mises au point à cet effet.

Dans les années 2000, une méthode d'amplification a été développée pour remplacer l'amplification en chaîne par polymérase (ou PCR pour « Polymerase Chain Reaction ») dans le cadre de l'amplification d'acides nucléiques. Cette méthode appelée LAMP et brevetée **[1]** présente l'avantage majeur d'être isotherme, l'amplification étant réalisée à une température constante typiquement comprise entre 60°C et 65°C, et permettant une détection rapide.

Plusieurs tests de diagnostic utilisant la méthode LAMP sont disponibles sur le marché pour des applications médicales, environnementales ou bien agro-agri alimentaires. Ces tests sont utilisés pour la détection d'ADN ou d'ARN génomique de virus, pathogènes ou autre **[2],** pour la détection de miARN **[3,4],** ou bien pour la détection de biomarqueurs **[5].**

Lors d'un test de diagnostic par LAMP, l'échantillon à analyser passe par plusieurs étapes avant la détection :
a. le prélèvement de l'échantillon,
b. l'extraction à partir de la matrice complexe des cibles d'intérêt protéique/ADN/ARN,
c. la mise en contact avec le mélange d'amplification LAMP contenant des amorces, des enzymes, des désoxyribonucléosides (dNTP) et un tampon d'amplification, et
d. le chauffage isotherme et le suivi de la production des amplicons produits par la LAMP au cours du temps généralement de l'ordre d'une heure.

Plusieurs de ces étapes peuvent présenter des problèmes amenant un test à se présenter comme un faux négatif, et il est difficile pour l'homme du métier d'identifier quelle étape est déficiente.

Une méthode proposant un indicateur permettant de vérifier que l'amplification isotherme (étapes c et d) se déroule correctement consiste à ajouter un contrôle interne dans la solution à amplifier. Ce contrôle est une cible oligonucléotidique différente de celle que l'on cherche à détecter qui s'amplifiera et qui sera le témoin du bon déroulement du test.

La manière la plus simple de mettre en place un contrôle interne dans les tests LAMP consiste à utiliser des méthodes de multiplexage pour détecter plusieurs cibles, une cible étant remplacée par le contrôle interne pour s'assurer du bon déroulement du test. Dans le cas des tests de détection PCR, la littérature scientifique sur le multiplexage est très abondante, mais beaucoup moins lorsqu'il s'agit des tests LAMP. Pour ces derniers, le multiplexage se réalise principalement en utilisant des couples fluorophore/quencher sur les amorces avec des longueurs d'ondes différentes de la cible à détecter **[6].**

En 2012, Tanner *et al* ont proposé une méthode de multiplexage en LAMP et décrit un procédé de DARQ LAMP pour le multiplexage **[7].** Les séquences décrites pourraient être utilisées pour réaliser un contrôle interne, mais la multiplication du nombre d'amorces en solution entraîne alors une augmentation du signal non spécifique. En effet, à chaque ajout de séquence, il faut s'assurer que les nouvelles amorces ajoutées ne génèrent pas de croisement avec les précédentes par des hybridations non spécifiques. De plus, chaque ajout de séquence produit un surcoût pour le test.

En 2022, Quyen *et al* ont proposé une méthode de multiplexage avec une première étape consistant en une LAMP suivie d'une deuxième étape d'amplification en phase solide **[8].** Les amorces utilisées pour amplifier les amplicons obtenus suite à la LAMP sont fixées sur un support solide moyennant quoi un produit d'amplification de type ADN double-brin marqué avec un colorant fluorescent du type cyanine 3 se trouve indirectement fixé sur le support solide. Dans cette méthode, la séquence servant de matrice à la LAMP et pouvant servir de contrôle interne est une séquence double-brin relativement longue : pour l'un des brins, on trouve une séquence B3 à l'extrémité 5' et une séquence F3c à l'extrémité 3' et, pour l'autre brin, une séquence F3 à l'extrémité 5' et une séquence B3c à l'extrémité 3'. L'amplification de cette matrice lors de la 1^{ère} étape de la LAMP nécessite quatre amorces à savoir une amorce F3, une amorce B3, une amorce FIP et une amorce BIP marquée avec une cyanine 3. Enfin d'autres amorces sont encore nécessaires pour poursuivre la LAMP : amorces FIP et BIP modifiées. Suite à cette amplification, des oligonucléotides à double structure tige-boucle sont générés : ils n'ont pas été synthétisés préalablement à toute amplification et, pour certains, sont marqués avec une cyanine 3. Là encore, la méthode proposée présente un coût non négligeable la rendant difficilement exploitable pour une amplification LAMP avec contrôle interne.

Il existe donc un réel besoin de proposer des contrôles internes systématiques dans les tests de diagnostic par LAMP afin de réduire les cas de faux négatifs. Cependant actuellement, très peu de dispositifs commerciaux de diagnostic par LAMP mettent en oeuvre un tel contrôle interne car celui-ci est complexe et coûteux à mettre en oeuvre.

Les inventeurs se sont donc fixé pour but de proposer un contrôle interne simple, peu coûteux, ne nécessitant pas un nombre important d'éléments tels que des amorces et, de fait, ne présentant pas ou peu de signal non spécifique.

### EXPOSÉ DE L'INVENTION

La présente invention permet d'atteindre le but que se sont fixé les inventeurs. En effet, ces derniers ont pu mettre au point un oligonucléotide simple-brin ci-après désigné « oligonucléotide contrôle » combiné uniquement à deux amorces nécessaires à son amplification et éventuellement une troisième séquence utile pour le suivi de cette amplification, comme contrôle interne dans une LAMP et ne présentant pas les inconvénients des contrôles internes actuellement proposés sur le marché ou décrits dans la littérature pour une telle amplification.

L'oligonucléotide contrôle mis en oeuvre dans l'invention a la particularité de ne nécessiter que deux amorces pour son amplification isotherme. Cette réduction du nombre d'amorces présente de nombreux avantages comme une simplicité de mise en oeuvre et un gain en termes de coût du fait que les séquences sont non seulement peu nombreuses mais aussi courtes. Le signal non spécifique est également réduit du fait de la réduction du nombre d'amorces.

En effet, cet oligonucléotide contrôle est relativement court ce qui en facilite la synthèse chimique mais aussi ce qui réduit la formation de structures chimiques secondaires et l'amplification non spécifique avec les amorces nécessaires à l'amplification de l'échantillon.

Par conséquent, cet oligonucléotide contrôle et au plus trois amorces ou séquences utiles à son amplification et/ou à la détection de cette amplification sont faciles à intégrer, en tant que contrôle interne, dans les tests LAMP. La séquence de l'oligonucléotide contrôle est générique : il peut être utilisé conjointement à de nombreuses cibles dans les échantillons. En effet, il a plus de chance de convenir et de ne pas croiser avec un grand nombre d'échantillons.

La présente invention concerne donc un procédé pour contrôler la qualité d'une amplification isotherme médiée par des boucles d'un mélange réactionnel contenant un contrôle interne et éventuellement au moins une séquence nucléotidique d'intérêt, ledit procédé comprenant
a) la co-amplification isotherme médiée par des boucles dudit contrôle interne et de ladite séquence nucléotidique d'intérêt éventuellement présente dans ledit mélange réactionnel et
b) le contrôle de la qualité de l'amplification isotherme médiée par des boucles en détectant le produit de l'amplification dudit contrôle interne,
caractérisé en ce que ledit contrôle interne se présente sous forme d'un oligonucléotide de formule (I) :

(F1c)ₘ-F2-(F0')ₙ-F1-(Int)ₚ-B1c-(B0')_{q}-B2c-(B1)ᵣ (I)

dans laquelle
m, n, p, q et r, identiques ou différents, sont égaux à 0 ou 1,
la portion F2 comprend de 8 à 30 nucléotides,
la portion F1 comprend de 10 à 35 nucléotides,

Int séparant la portion F1 et la portion B1c est, lorsque p est égal à 0, une liaison covalente et, lorsque p est égal à 1, une portion comprenant au moins un nucléotide,
la portion B1c comprend de 10 à 35 nucléotides,
la portion B2c comprend de 8 à 35 nucléotides,
F1c représente, lorsque m est égal à 0, l'extrémité 5' de la portion F2 et, lorsque m est égal à 1, une portion dont la séquence nucléotidique est complémentaire à la séquence nucléotidique de la portion F1,
F0' représente, lorsque n est égal à 0, une liaison covalente liant les portions F2 et F1 et, lorsque n est égal à 1, une portion comprenant au moins un nucléotide,
B0' représente, lorsque q est égal à 0, une liaison covalente liant les portions B1c et B2c et, lorsque q est égal à 1, une portion comprenant au moins un nucléotide, et
B1 représente, lorsque r est égal à 0, l'extrémité 3' de la portion B2c et, lorsque r est égal à 1, une portion dont la séquence nucléotidique est complémentaire à la séquence nucléotidique de la portion B1c et
en ce que ledit mélange réactionnel contient au plus deux amorces utiles pour l'amplification isotherme médiée par des boucles dudit contrôle interne et éventuellement une autre séquence nucléotidique pour la détection de l'amplification dudit contrôle interne.

Par « contrôler la qualité d'une amplification isotherme médiée par des boucles », on entend vérifier la bonne préparation de l'échantillon à détecter et celle du mélange réactionnel (enzyme, dNTPs, tampons, etc...), la fonctionnalité de tous les composants utilisés pour cette amplification, l'absence de facteurs inhibant cette dernière et/ou l'absence d'éléments dégradant les produits d'amplification.

Dans ce qui suit et ce qui précède, les expressions « oligonucléotide mis en oeuvre dans l'invention », « oligonucléotide contrôle », « contrôle interne » et « oligonucléotide de formule (I) » sont équivalentes et utilisables de façon interchangeable.

Tout d'abord, il convient de noter qu'il est facile de préparer l'oligonucléotide mis en oeuvre dans l'invention à partir de la séquence de toutes amorces FIP (pour « Forward Inner Primer ») de séquence 5'-F1c-F2-3' et BIP (pour « Backward Inner Primer ») de séquence 5'-B1c-B2-3' décrites dans l'art antérieur. En effet, cet oligonucléotide comprend une portion F2 également présente dans l'amorce FIP, une portion F1 complémentaire à la portion F1c présente dans l'amorce FIP, une portion B1c également présente dans l'amorce BIP et une portion B2c complémentaire à la portion B2 présente dans l'amorce BIP, ledit oligonucléotide pouvant, en outre, contenir une portion F1c et une portion B1. Dans certaines formes de mises en oeuvre, l'oligonucléotide peut présenter d'autres séquences non liées à FIP et à BIP telles que les séquences B0', F0' et Int.

Dans le cadre de la présente invention, deux séquences nucléotidiques sont complémentaires l'une de l'autre, lorsqu'un nombre suffisant de nucléotides de la première séquence nucléotidique peut se lier, au moyen de liaisons hydrogène, avec les nucléotides correspondants de la deuxième séquence nucléotidique de telle sorte que l'appariement entre les deux séquences nucléotidiques peut se produire. La « complémentarité », telle qu'utilisée ici, se réfère à la capacité d'appariement entre les nucléotides d'une première séquence nucléotidique et d'une seconde séquence nucléotidique. Des nucléotides non complémentaires entre deux séquences nucléotidiques peuvent être tolérés à condition que les deux séquences nucléotidiques restent capables de s'hybrider spécifiquement l'une à l'autre. De plus, une première séquence nucléotidique peut s'hybrider sur un ou plusieurs segments d'une seconde séquence nucléotidique de telle sorte que les segments intermédiaires ou adjacents ne sont pas impliqués dans l'hybridation. Dans certaines formes de mise en oeuvre de l'invention, une première séquence nucléotidique ou une partie spécifique de celle-ci, est au moins 70%, 80%, 85%, 86%, 87%, 88%, 89%, 90%, 91%, 92%, 93%, 94%, 95%, 96%, 97%, 98%, 99% ou 100% complémentaire à une deuxième séquence nucléotidique ou une partie spécifiée de celle-ci.

Typiquement, dans l'oligonucléotide mis en oeuvre dans l'invention, la portion F2 peut comprendre de 10 à 27 nucléotides et, à titre d'exemples particuliers, 13, 14, 15, 16, 17, 18, 19, 20, 21, 22, 23 et 24 nucléotides.

Typiquement, dans l'oligonucléotide mis en oeuvre dans l'invention, la portion F1 peut comprendre de 15 à 25 nucléotides et, à titre d'exemples particuliers, 19, 20, 21 ou 22 nucléotides.

Typiquement, dans l'oligonucléotide mis en oeuvre dans l'invention, la portion B1c peut comprendre de 17 à 27 nucléotides et, à titre d'exemples particuliers, 18, 19, 20, 21, 22, 23, 24 ou 25 nucléotides.

Typiquement, dans l'oligonucléotide mis en oeuvre dans l'invention, la portion B2c peut comprendre de 15 à 30 nucléotides et, à titre d'exemples particuliers, 18, 19, 20, 21 ou 22 nucléotides.

Typiquement, dans l'oligonucléotide mis en oeuvre dans l'invention, lorsque Int représente une portion comprenant au moins un nucléotide, cette dernière peut comprendre jusqu'à 3 nucléotides, 10 nucléotides, 30 nucléotides, 50 nucléotides et voire 70 nucléotides.

Il est évident que, pour servir de contrôle interne, il faut que l'oligonucléotide mis en oeuvre dans l'invention ne contienne aucune séquence capable de se lier, directement ou indirectement, à l'analyte d'intérêt et/ou à la séquence nucléotidique d'intérêt susceptible d'être détecté(e), directement ou indirectement, par l'amplification LAMP dont on souhaite contrôler la qualité. Par ailleurs, lorsque la distinction entre l'amplification LAMP de l'oligonucléotide contrôle et celle de la séquence nucléotidique d'intérêt ne se fait pas dans l'espace (fixation de l'oligonucléotide de contrôle sur un support solide) ou dans le temps (vitesse d'amplification de l'oligonucléotide contrôle et celle de la séquence nucléotidique d'intérêt distinctes), l'oligonucléotide contrôle ne doit contenir aucune séquence capable de se lier aux amorces utilisées pour l'amplification LAMP de la séquence nucléotidique d'intérêt.

Dans une première forme de mise en oeuvre qui correspond au cas où m, n, q et r sont tous égaux à 0, l'oligonucléotide utilisé dans l'invention répond à la formule (II) ci-après :

5'-F2-F1-(Int)ₚ-B1c-B2c-3' (II)

avec p et les portions F2, F1, Int, B1c et B2c tels que précédemment définis.

L'oligonucléotide de formule (II) ne présente aucune structure secondaire interne i.e. la formule (II) ne présente aucune séquence d'au moins 4 nucléotides consécutifs complémentaires à une autre séquence dans ladite formule (II). On peut parler d'oligonucléotide linéaire.

L'extrémité 5' de l'oligonucléotide de formule (II) correspond à l'extrémité 5' de la portion F2 et son extrémité 3' à l'extrémité 3' de la portion B2c.

Avantageusement, l'oligonucléotide de formule (II) utilisé dans l'invention peut comprendre moins de 130 nucléotides, notamment moins de 120 nucléotides, en particulier moins de 110 nucléotides, plus particulièrement moins de 100 nucléotides et tout particulièrement moins de 90 nucléotides.

Avec une telle taille, la synthèse chimique de l'oligonucléotide de formule (II) est simple, rapide et le coût de cette synthèse et, par conséquent, le coût du contrôle interne pour une amplification LAMP sont moindres. Cependant son temps d'amplification peut être considéré comme lent (environ 45 min). L'oligonucléotide de formule (II) est à privilégier pour des cas où le coût est une considération plus importante que le temps d'amplification.

Dans une seconde forme de mise oeuvre qui correspond au cas où m, n, q et r sont tous égaux à 1, l'oligonucléotide utilisé dans l'invention présente une double structure tige-boucle et répond à la formule (III) ci-après :

5'-F1c-F2-F0'-F1-(Int)ₚ-B1c-B0'-B2c-B1-3' (III)

avec p et les portions F1c, F2, F0', F1, Int, B1c, B0', B2c et B1 tels que précédemment définis.

L'extrémité 5' de l'oligonucléotide de formule (III) correspond à l'extrémité 5' de la portion F1c et son extrémité 3' à l'extrémité 3' de la portion B1.

Dans l'oligonucléotide de formule (III) mis en oeuvre dans l'invention, la portion F1c et la portion F1 s'hybrident pour former la tige de la première structure tige-boucle de cet oligonucléotide. Typiquement, dans l'oligonucléotide à double structure tige-boucle de formule (III) mis en oeuvre dans l'invention, la portion F1 et la portion F1c comprennent, chacune, de 10 à 35 nucléotides, notamment entre 15 et 25 nucléotides et, à titre d'exemples particuliers, 19, 20, 21 ou 22 nucléotides. La température de fusion (Tm) de F1-F1c est notamment supérieure à la température utilisée lors de l'amplification LAMP et, en particulier, supérieure de 5°C à 7°C. L'homme du métier pourra trouver des informations additionnelles quant à ces portions dans **[1].**

Dans l'oligonucléotide de formule (III) mis en oeuvre dans l'invention, la portion F2-F0' forme la boucle de la première structure tige-boucle, désignée ci-après boucle F0. Typiquement, la portion F2 comprend de 8 à 30 nucléotides, notamment de 10 à 27 nucléotides et, à titre d'exemples particuliers, 13, 14, 15, 16, 17, 18, 19, 20, 21, 22, 23 et 24 nucléotides et la portion F0' est telle que la boucle F0 comprend de 10 à 70 nucléotides.

Dans l'oligonucléotide de formule (III) mis en oeuvre dans l'invention, la portion B1 et la portion B1c s'hybrident pour former la tige de la seconde structure tige-boucle. Typiquement, dans l'oligonucléotide à double structure tige-boucle de formule (III) mis en oeuvre dans l'invention, la portion B1 et la portion B1c comprennent, chacune, de 10 à 35 nucléotides, notamment entre 17 à 27 nucléotides et, à titre d'exemples particuliers, 18, 19, 20, 21, 22, 23, 24 ou 25 nucléotides. La température Tm de B1-B1c est notamment supérieure à la température utilisée lors de l'amplification et, en particulier, supérieure de 5°C à 7°C. L'homme du métier pourra trouver des informations additionnelles quant à ces portions dans **[1].**

Dans l'oligonucléotide de formule (III) mis en oeuvre dans l'invention, la portion B0'-B2c forme la boucle de la seconde structure tige-boucle, désignée ci-après boucle B0. De plus, la portion B2c comprend de 8 à 35 nucléotides, notamment de 15 à 30 nucléotides et, à titre d'exemples particuliers, 18, 19, 20, 21 ou 22 nucléotides et la portion B0' est telle que la boucle B0 comprend de 10 à 70 nucléotides.

Même si l'oligonucléotide de formule (III) mis en oeuvre dans l'invention est plus long et donc plus coûteux et complexe à synthétiser chimiquement que l'oligonucléotide de formule (II), sa vitesse d'amplification est nettement plus rapide que celle de l'oligonucléotide de formule (II) (environ 15 min). L'oligonucléotide de formule (III) est donc à privilégier pour les cas où la rapidité du test est prévalente par rapport à son coût.

L'oligonucléotide de formule (III) mis en oeuvre dans l'invention est clairement différent des oligonucléotides à double structure tige-boucle décrits dans **[8]** puisque ces derniers sont préparés durant l'amplification LAMP.

Dans une troisième forme de mise en oeuvre qui correspond au cas où m est égal à 0 et n, q et r sont égaux à 1, l'oligonucléotide utilisé dans l'invention présente une seule structure tige-boucle et répond à la formule (IV) ci-après :

5'-F2-F0'-F1-(Int)p-B1c-B0'-B2c-B1-3' (IV)

avec p et les portions F2, F0', F1, Int, B1c, B0', B2c et B1 tels que précédemment définis.

L'extrémité 5' de l'oligonucléotide de formule (IV) correspond à l'extrémité 5' de la portion F2 et son extrémité 3' à l'extrémité 3' de la portion B1.

Dans une quatrième forme de mise en oeuvre qui correspond au cas où r est égal à 0 et m, n et q sont égaux à 1, l'oligonucléotide utilisé dans l'invention présente une seule structure tige-boucle et répond à la formule (V) ci-après :

5'-F1c-F2-F0'-F1-(Int)ₚ-B1c-B0'-B2c-3' (V)

avec p et les portions F1c, F2, F0', F1, Int, B1c, B0' et B2c tels que précédemment définis.

L'extrémité 5' de l'oligonucléotide de formule (V) correspond à l'extrémité 5' de la portion F1c et son extrémité 3' à l'extrémité 3' de la portion B2c.

Les oligonucléotides contrôles de formule (IV) ou (V) constituent des intermédiaires à ceux de formule (II) et (III). En effet, ils sont plus longs et donc plus coûteux que ceux de formule (II) mais plus courts et donc moins coûteux que ceux de formule (III) et présentent une vitesse d'amplification plus élevée que celle observée pour les oligonucléotides de formule (II).

Dans le cadre de l'invention, l'oligonucléotide de formule (I) et donc les oligonucléotides de formule (II), (III), (IV) et (V) sont synthétisés préalablement à leur introduction dans le mélange réactionnel et donc préalablement à toute étape d'amplification LAMP. Par ailleurs, la préparation des oligonucléotides à double structure tige-boucle décrits dans **[8]** nécessitent a minima quatre amorces (F3, B3, FIP et BIP-cy3).

Pour que l'oligonucléotide mis en oeuvre soit un contrôle interne pertinent, ce dernier et l'éventuelle séquence nucléotidique d'intérêt doivent être dans un même mélange réactionnel et réaliser l'amplification LAMP de cet oligonucléotide et de cette séquence nucléotidique d'intérêt dans un même volume de ce mélange. Ce volume peut être le volume de mélange réactionnel contenu dans une chambre réactionnelle unique ou le volume d'une goutte de ce mélange réactionnel. De cette façon, l'amplification de l'oligonucléotide et de la séquence nucléotidique d'intérêt, si présente, se fait dans les mêmes conditions en termes de composants du mélange réactionnel et de température.

Dans le cadre du procédé de contrôle selon l'invention, l'oligonucléotide mis en oeuvre peut se trouver en solution dans le mélange réactionnel.

En variante, il peut être fixé au niveau de la surface d'un support solide avec lequel le mélange réactionnel est en contact. Ce support solide peut être, à titre d'exemples illustratifs, une plaque, un support de biopuce, la surface interne d'un microtube, la surface interne d'un puits d'une plaque multipuits ou encore la surface d'une bille éventuellement magnétique. Ce support solide peut être en silicium, en verre, en métal, en polymère, en latex ou en plastique.

Dans une forme de mise en oeuvre particulière, la surface du support solide présente des groupements fonctionnels grâce auxquels l'oligonucléotide mis en oeuvre est capable d'être immobilisé. De façon avantageuse, ces groupements fonctionnels sont choisis parmi les groupements carboxyliques, des entités radicalaires, les fonctions alcools, amines, amides, époxy ou thiols. Ces groupements sont portés, de façon intrinsèque ou par fonctionnalisation, par la surface du support solide. L'oligonucléotide mis en oeuvre peut également présenter de tels groupements fonctionnels, de façon intrinsèque ou par fonctionnalisation.

Dans une première variante de la présente invention, l'oligonucléotide peut être immobilisé de façon directe au niveau de la surface du support solide, fonctionnalisée ou non. Un support solide « coaté » avec une protéine telle que la streptavidine est un exemple d'immobilisation directe dans lequel l'oligonucléotide mis en oeuvre doit être fonctionnalisé par une biotine.

Dans une seconde variante de la présente invention, l'oligonucléotide peut être immobilisé de façon indirecte au niveau de la surface du support solide, fonctionnalisée ou non. Cette immobilisation indirecte fait intervenir un bras espaceur (ou agent de jonction) lié, d'une part, à la surface du support solide et, d'autre part, à un oligonucléotide tel que précédemment défini. Un tel bras espaceur est notamment utilisé pour améliorer l'accessibilité à l'oligonucléotide mis en oeuvre. A titre d'exemples illustratifs, on peut citer les réactifs silanes mis en oeuvre pour le greffage sur du verre, la complexation de produits thiolés sur des surfaces d'or et l'immobilisation de sondes dans des matrices polymères. En vue de la fixation indirecte de l'oligonucléotide mis en oeuvre, un tel bras espaceur peut se présenter sous forme d'une séquence comprenant plusieurs bases thymine et notamment 10 bases thymine.

Cette immobilisation indirecte peut également faire intervenir, en tant que bras espaceur, une séquence nucléotidique complémentaire à une partie de la séquence nucléotidique de l'oligonucléotide contrôle et fixée à la surface du support solide. Une telle séquence permet la fixation par hybridation, typiquement stable à une température supérieure à la température de la LAMP, de l'oligonucléotide contrôle. Une amorce FIP ou une amorce BIP fixée à la surface du support est un exemple particulier de ces séquences nucléotidiques complémentaires.

Les liaisons mises en oeuvre au cours d'une immobilisation directe ou indirecte peuvent être toutes liaisons connues de l'homme du métier et notamment des liaisons covalentes, des liaisons ioniques, des liaisons hydrogène, des interactions électrostatiques, des interactions hydrophobes, des liaisons de Van der Waals ou une adsorption.

Par « mélange réactionnel » on entend un mélange contenant tous les éléments nécessaires à une amplification LAMP ainsi que l'oligonucléotide contrôle tel que précédemment défini et les amorces ou séquences utiles à son amplification et à la détection de cette amplification et éventuellement une séquence nucléotidique d'intérêt.

L'oligonucléotide contrôle est présent dans le mélange réactionnel en une quantité comprise entre 1 aM et 1 mM, et notamment entre 100 aM et 1 µM). A titre d'exemples particuliers de concentrations utilisables, on peut citer une concentration de 10 pM, de 100 pM, de 1 nM, ou de 10 nM. Avantageusement, l'oligonucléotide contrôle est présent dans la solution mise en oeuvre lors de l'étape ii) à une concentration de 100 pM.

Comme précédemment indiqué, le mélange réactionnel mis en oeuvre dans le procédé d'amplification LAMP contrôlée comprend au plus deux amorces pour l'amplification et éventuellement une troisième séquence pour la détection de l'amplification du contrôle interne. Il est évident que ce mélange réactionnel peut contenir d'autres amorces impliquées dans l'amplification de la séquence nucléotidique d'intérêt. En variante, les deux amorces utilisées pour amplifier l'oligonucléotide contrôle et éventuellement la séquence pour détecter cette amplification peuvent également être utilisées pour l'amplification de la séquence nucléotidique d'intérêt et la détection de cette amplification. Cette variante peut s'appliquer au cas où la distinction entre amplification de l'oligonucléotide contrôle et détection de cette amplification et celles de la séquence nucléotidique d'intérêt se fait spatialement i.e. par une localisation contrôlée de l'un et/ou l'autre comme la fixation de l'oligonucléotide contrôle au niveau de la surface d'un support solide précédemment décrite.

Dans une première forme de mise en oeuvre, le mélange réactionnel comprend, comme seules amorces impliquées dans l'amplification de l'oligonucléotide contrôle et la détection de cette amplification, les amorces FIP et BIP telles que précédemment définies.

Dans une seconde forme de mise en oeuvre, le mélange réactionnel comprend, comme seules amorces impliquées dans l'amplification de l'oligonucléotide contrôle et la détection de cette amplification, les amorces FIP et BIP telles que précédemment définies, l'une des deux étant marquée, à son extrémité 5' ou 3', par un premier fluorochrome, et une troisième séquence nucléotidique marquée par un second fluorochrome. Cette troisième séquence nucléotidique est en partie complémentaire à la séquence nucléotidique de l'extrémité de l'amorce FIP ou de BIP marquée par le premier fluorochrome. L'un des deux entre le premier et le second fluorochromes est un fluorochrome émetteur (ou « reporter ») comme, par exemple, la 6-carboxyfluorescéine (6-FAM), la tetrachlorofluorescéine (TET), l'hexachlorofluorescéine (HEX), la cyanine 3 (Cy3) ou la cyanine 5 (Cy5) et l'autre est un fluorochrome suppresseur (ou « quencher ») comme, par exemple, la 6-carboxy-tetraméthyl-rhodamin (TAMRA), le BHQ1 (pour « Black Hole Quencher 1 »), le BHQ2 ou le BHQ3). Lorsque stimulé, le fluorochrome émetteur transfère son énergie au fluorochrome suppresseur voisin par le principe de FRET (pour « Fluorescence Resonance Energy Transfer ») qui dissipe cette énergie sous forme de chaleur. Durant l'amplification LAMP, le fluorochrome émetteur est alors libéré de l'environnement du fluorochrome suppresseur permettant ainsi l'émission de fluorescence. Dans un exemple particulier de cette seconde forme de mise en oeuvre, le mélange réactionnel comprend une amorce FIP, une amorce BIP dont l'extrémité 5' est marquée par un fluorochrome émetteur et une troisième séquence nucléotidique présentant, à son extrémité 3', une portion B1 (dont la séquence nucléotidique est complémentaire à la séquence nucléotidique de la portion B1c présente à l'extrémité 5' de BIP) et un fluorochrome suppresseur.

Typiquement, le mélange réactionnel mis en oeuvre comprend un tampon approprié, comme, par exemple, un tampon phosphate ou un tampon Tris; des désoxyribonucléosides (dNTP), comme, un mélange équimolaire de dATP, dCTP, dGTP et dTTP, des sels comme des sels de magnésium tels que MgSO₄ et MgCl₂, des sels de manganèse tels que MnCl₂, des sels de potassium tels que KCI et/ou des sels d'ammonium ; des détergents comme du Tween^{®} 20, de la bétaïne et une enzyme catalysant l'amplification LAMP. Une telle enzyme est une polymérase présentant une activité de déplacement de brin élevée en plus d'une activité de réplication. A titre d'exemples particuliers de telles enzymes, on peut utiliser l'une des enzymes suivantes : Bst DNA polymerase et ses variantes telles que, par exemple, Bst2.0 DNA polymerase et Bst3.0 DNA polymerase ; Bsm DNA polymerase ; Bca (exo-) DNA polymerase ; Klenow fragment of DNA polymerase I ; Vent DNA polymerase ; Vent(Exo-) DNA polymerase (exonuclease activity-free Vent DNA polymerase) ; DeepVent DNA polymerase ; DeepVent(Exo-) DNA polymerase (DeepVent DNA polymerase sans activité exonucléase) ; 29 phage DNA polymerase ; MS-2 phage DNA polymerase ; Z-Taq DNA polymerase (Takara Shuzo), et KOD DNA polymerase (TOYOBO).

Le mélange réactionnel peut comprendre, en outre, des éléments utiles pour la détection et la quantification du produit d'amplification de l'oligonucléotide contrôle ou de la séquence nucléotidique d'intérêt éventuellement présente dans le mélange réactionnel comme, par exemple, de la calcéine, un colorant intercalant comme, par exemple, iodure de propidium, SYTO 9, le vert SYBR et l'EvaGreen.

L'expression « séquence nucléotidique » utilisée dans la présente est équivalente aux termes et expressions suivants : « acide nucléique », « polynucléotide », « molécule nucléotidique », « séquence polynucléotidique » et « séquence oligonucléotidique ». Par « séquence nucléotidique », on entend, dans le cadre de la présente invention, un chromosome ; un gène ; un polynucléotide régulateur; un ADN, simple-brin ou double-brin, génomique, chromosomique, chloroplastique, plasmidique, mitochondrial, recombinant ou complémentaire ; un ARN total ; un ARN messager; un ARN ribosomal (ou ribozyme) ; un ARN de transfert ; un micro-ARN ; un petit ARN interférent ; une séquence faisant office d'aptamère ; un acide nucléique peptidique ; un acide nucléique verrouillé (ou LNA pour « Locked Nucleic Acid ») ; un morpholino ; un oligonucléotide à double structure tige boucle une portion ou un fragment de ceux-ci.

Dans une première forme de mise en oeuvre, la séquence nucléotidique d'intérêt peut être l'analyte cible que l'on cherche à détecter et éventuellement à quantifier via le procédé d'amplification contrôlée selon l'invention.

Dans une seconde forme de mise en oeuvre, la séquence nucléotidique d'intérêt peut être une séquence permettant de détecter et éventuellement quantifier l'analyte cible de façon indirecte. Cette forme de mise en oeuvre s'applique notamment lorsque l'analyte cible n'est pas de nature nucléotidique ou ne comprend pas de séquence nucléotidique. Ainsi l'analyte cible à détecter peut être choisi dans le groupe constitué par une molécule d'intérêt environnemental tel qu'un pesticide ; une molécule d'intérêt biologique ; une molécule d'intérêt pharmacologique ; une toxine ; un glucide tel que du glucose ; un lipide tel que du cholestérol ; un peptide ; un antigène ; un épitope ; une protéine ; une glycoprotéine ; une enzyme ; un substrat enzymatique ; un récepteur nucléaire ou membranaire ; un agoniste ou un antagoniste d'un récepteur nucléaire ou membranaire ; une hormone ; un anticorps polyclonal ou monoclonal ; un fragment d'anticorps tel qu'un fragment Fab, F(ab')₂, Fv, scFv ou un domaine hypervariable (ou CDR pour « Complementarity Determining Region ») ; des ions tels que des ions mercure ou des ions plomb ; une cellule eucaryote ; une cellule procaryote et un virus.

Dans cette seconde forme de mise en oeuvre, la séquence nucléotidique d'intérêt présente au moins une entité capable de se lier, directement ou indirectement, à l'analyte cible. Pour cette forme de mise en oeuvre, il est possible d'utiliser, comme séquence nucléotidique d'intérêt, un oligonucléotide à double structure tige-boucle tel que décrit dans les demandes de brevet EP 3878971 et EP 22195865.5 **[9,10].**

A noter que, dans les première et seconde formes de mise en oeuvre, un analyte cible peut être défini comme une petite molécule i.e. une molécule dont le poids moléculaire est inférieur ou égal à 10000 daltons et notamment inférieur ou égal à 8000 daltons. Cette petite molécule peut appartenir à l'un quelconque des éléments des listes d'analytes ci-dessus.

Dans le procédé d'amplification LAMP contrôlée selon l'invention, l'étape de co-amplification est mise en oeuvre pendant une période de temps et à une température suffisantes pour la production d'un produit d'amplification à partir de l'oligonucléotide contrôle et de la séquence nucléotidique d'intérêt, si présente. Avantageusement, cette étape est effectuée dans des conditions isothermes et notamment à une température comprise entre 15°C et 90°C, en particulier, comprise entre 50°C et 80°C et, plus particulièrement de l'ordre de 65°C (i.e. 65°C ± 5°C). La durée de cette étape de co-amplification selon l'invention est comprise entre 1 min et 120 min, notamment entre 3 min et 90 min et, en particulier, entre 5 min et 60 min.

Dans le procédé d'amplification LAMP contrôlée selon l'invention, ce contrôle est réalisé en détectant les produits d'amplification de l'oligonucléotide contrôle. Cette détection peut être réalisée en même temps que l'amplification LAMP ou après cette dernière.

Cette détection des produits d'amplification dudit oligonucléotide peut se faire :
- par mesure de la fluorescence,
- en détectant les sous-produits de la réaction d'amplification comme, par exemple, des cristaux de pyrophosphate, ou
- par mesure de la variation de pH.

Il est évident que cette détection ne doit pas interférer avec la détection des produits d'amplification de la séquence nucléotidique d'intérêt.

Lorsque l'oligonucléotide contrôle est en solution, la détection peut se faire en suivant la libération de la fluorescence de l'un des fluorophores portés par l'amorce FIP, l'amorce BIP ou la troisième séquence nucléotidique telle que précédemment définie.

La distinction peut également se faire via la fixation de l'oligonucléotide contrôle au niveau d'une première zone de la surface du support solide, la séquence nucléotidique d'intérêt et/ou l'analyte cible pouvant, quant à eux, être en solution ou fixés, directement ou indirectement, au niveau d'une seconde zone de la surface du support solide, distincte de la première zone.

Dans ce cas, la détection peut être réalisée en détectant des cristaux en plan par imagerie à l'oeil nu, via un turbidimètre ou par imagerie sans lentille **[11].**

Dans ce cas, la détection peut également être réalisée par mesure de fluorescence lorsque la calcéine ou un colorant intercalant fluorescent ou encore une sonde marquée par un fluorochrome et reconnaissant le produit d'amplification est présent(e) dans le mélange réactionnel.

Dans ce cas, la détection peut également être réalisée en mesurant la variation de pH, par exemple, par colorimétrie, par électrochimie ou via une sonde pH et ce, afin de suivre l'acidification du mélange réactionnel lors de l'amplification LAMP.

Les différentes variantes envisagées lorsque l'oligonucléotide contrôle est fixé sur un support solide s'appliquent également au cas où l'amplification de l'oligonucléotide contrôle est suffisamment lente i.e. supérieure à 30 minutes pour ne pas interférer avec l'amplification de la séquence nucléotidique d'intérêt.

La présente invention concerne également un kit pour contrôler la qualité d'une amplification LAMP consistant en un oligonucléotide contrôle tel que précédemment défini et les au plus deux amorces pour son amplification et l'éventuelle troisième séquence nucléotidique pour la détection de cette amplification telles que précédemment définies.

En d'autres termes, la présente invention concerne :
- un kit pour contrôler la qualité d'une amplification LAMP consistant en un oligonucléotide contrôle tel que précédemment défini et les amorces FIP et BIP et
- un kit pour contrôler la qualité d'une amplification LAMP consistant en un oligonucléotide tel que précédemment défini, les amorces FIP et BIP, l'une des deux étant marquée, à son extrémité 5' ou 3', par un premier fluorochrome, et une troisième séquence nucléotidique marquée par un second fluorochrome.

D'autres caractéristiques et avantages de la présente invention apparaîtront encore à l'homme du métier à la lecture des exemples ci-après donnés à titre illustratif et non limitatif, en référence aux figures annexées.

### BRÈVE DESCRIPTION DES DESSINS

La Figure 1 présente un exemple de courbes d'amplification de la séquence 1 (oligonucléotide à double structure tige boucle de formule (III)) pour des concentrations de 1 nM, 100 pM et 10 pM.
La Figure 2 présente un exemple de courbes d'amplification de la séquence 3 (oligonucléotide linéaire de formule (II)) pour des concentrations de 1 nM, 100 pM et 10 pM.
La Figure 3 présente un exemple de courbes d'amplification de la séquence 5 (oligonucléotide à une seule tige boucle de formule (V)) pour des concentrations de 1 nM, 100 pM et 10 pM.
La Figure 4 présente les courbes d'amplification de la séquence 1 avec les amorces 1 (trait plein) et avec les amorces 2 (trait pointillé). Fluorescence (unité arbitraire) en fonction du temps.
La Figure 5 présente les courbes d'amplification de la séquence 2 avec les amorces 1 (trait pointillé) et avec les amorces 2 (trait plein). Fluorescence (unité arbitraire) en fonction du temps.
La Figure 6 présente les courbes d'amplification de la séquences 1 en présence de ses amorces (trait plein), ou d'une seule des amorces (rond et croix) ou d'aucune amorce (triangle).

### EXPOSÉ DÉTAILLÉ DE MODES DE RÉALISATION PARTICULIERS

### 1. Matériel et Méthodes.

Le Tableau 1 ci-après décrit la séquence nucléotidique des différents oligonucléotides contrôles selon l'invention ainsi que les amorces FIP et BIP utilisées pour leur amplification.

Un mode de réalisation possible du contrôle interne proposé ici consiste à préparer des solutions d'oligonucléotide avec, par exemple, les séquences décrites dans le Tableau 1 ci-après, à des concentrations différentes choisies selon l'efficacité de l'amplification voulue (concentrée pour rapidité) et à les amplifier selon le protocole d'amplification LAMP à 2 amorces tels que décrits dans [**9,10**].

Ce protocole consiste à ajouter 2 µL de solution d'oligonucléotide dilué dans un tampon contenant par exemple du PBS et MgClz avec 18 µL de mix réactionnel LAMP comprenant :
Un Master Mix commercial contenant une enzyme catalysant l'amplification (exemple NEB Warm Start LAMP kit), concentrations typiques 1X, 0,75X ou 0,5X finales. Un intercalent fluorescent tel que l'Eva Green est ajouté à des concentrations finales comprises entre 2X et 0.1X (typiquement 0.25X) et les amorces FIP et BIP à des concentrations comprises entre 0.1 et 5 µM (typiquement 2.4 µM).

### [Table 1]

**Tableau 1 : Séquences d'oligonucléotides pour les conceptions 1 et 2**

| | | |
|---|---|---|
| Conception 1 | Séquence 1 (Formule (III)) | 5′– GGTTGGTGTGGTTGGTTTTTTTTTTCGGATCCAAGACGCGGTTTA TCGTGCAGTACGCCAACCTTTCTCAATAAACCGCGTCTTGGATCC GTGACATCTCTGAGTTATATCTTTCCCCCCCTCACTGACCCTCCTT CGGGGGAAAGATATAACTCAGAGATG–3′ (SEQ ID NO: 1 dans le listage de séquences en annexe) |
| | Séquence 3 (Formule (II) | 5'- CGGCAGTACGCCAACCTTTCTCAATAAACCGCGTCTTGGATCCGC ATCTCTGAGTTATATCTTTCCCCCCCTCACTGACCCTCCTTC-3' (SEQ ID NO: 2 dans le listage de séquences en annexe) |
| | Séquence 4 (Formule (IV)) | 5' CGTGCAGTACGCCAACCTTTCTCAATAAACCGCGTCTTGGATCCG CATCTCTGAGTTATATCTTTCCCCCCCTCACTGACCCTCCTTCGGG GGAAAGATATAACTCAGAGATG-3' (SEQ ID NO: 3 dans le listage de séquences en annexe) |
| | Séquence 5 (Formule (V)) | 5'- CGGATCCAAGACGCGGTTTATCGTGCAGTACGCCAACCTTTCTCA ATAAACCGCGTCTTGGATCCGTGACATCTCTGAGTTATATCTTTCC CCCCCTCACTGACCCTCCTTC-3' (SEQ ID NO: 4 dans le listage de séquences en annexe) |
| | FIP1 | 5′- CGGATCCAAGACGCGGTTTATCGTGCAGTACGCCAACCTTTCTCA -3′ (SEQ ID NO: 5 dans le listage de séquences en annexe) |
| | BIP1 | 5′- CATCTCTGAGTTATATCTTTCCCCCGAAGGAGGGTCAGTGAGG-3′ (SEQ ID NO: 6 dans le listage de séquences en annexe) |
| Conception 2 | Séquence 2 (Formule (III)) | 5'- GGTTGGTGTGGTTGGTTTTTTTTTTAGAGCAGCAGAAGTGGCAC AGGTGATTGTGAAGAAGAAGAGTGTGCCACTTCTGCTGCTCTTT ATCAACCTGAAGAAGAGCAGGCAGTGAGGACAATCAGTTCTTGC TCTTCTTCAGGTTGA-3' (SEQ ID NO: 7 dans le listage de séquences en annexe) |
| | FIP2 | 5'- AGAGCAGCAGAAGTGGCACAGGTGATTGTGAAGAAGAAGAG-3' (SEQ ID NO: 8 dans le listage de séquences en annexe) |
| | BIP2 | 5'-TCAACCTGAAGAAGAGCAAGAACTGATTGTCCTCACTGCC-3' (SEQ ID NO: 9 dans le listage de séquences en annexe) |

### 2. Preuve de l'amplification des séquences proposées.

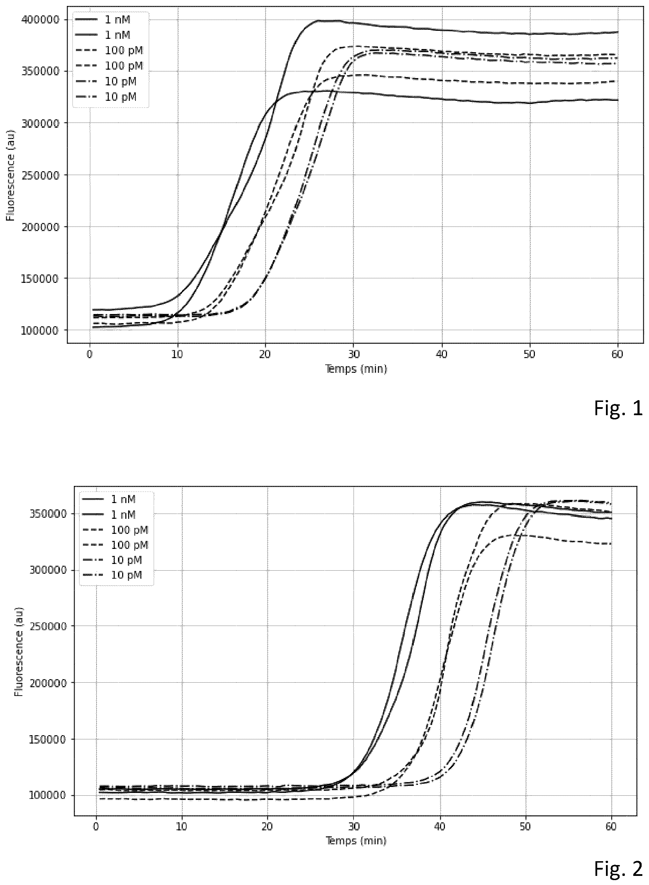

Différentes concentrations d'oligonucléotides (1 nM, 100 pM, 10 pM), sont mises en contact avec le mix d'amplification décrit ci-dessus, et la fluorescence est suivie en fonction du temps (intercalant) afin de suivre la formation d'amplicons. Les Figure 1 à 3 représentent respectivement les courbes d'amplification pour les séquences 1, 3 et 5 telles que définies dans le Tableau 1 pour différentes concentrations d'oligonucléotides en solution, validant ainsi la fonctionnalité de ces séquences.

### 3. Preuve de la sélectivité des séquences proposées.

Afin de prouver la sélectivité des séquences proposées, deux séquences (séquences 1 et 2) sont conçues comprenant toutes les deux leurs amorces spécifiques (FIP1 et BIP1, d'une part, et FIP2 et BIP2, d'autre part). Ces deux séquences sont mises en contact avec diverses amorces pour tester la sélectivité de l'amplification.

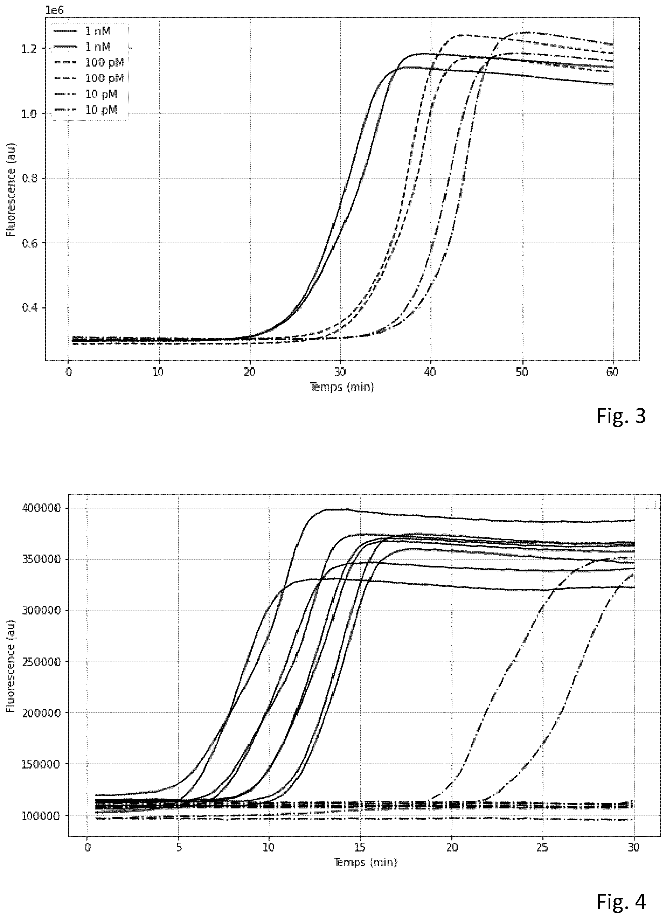

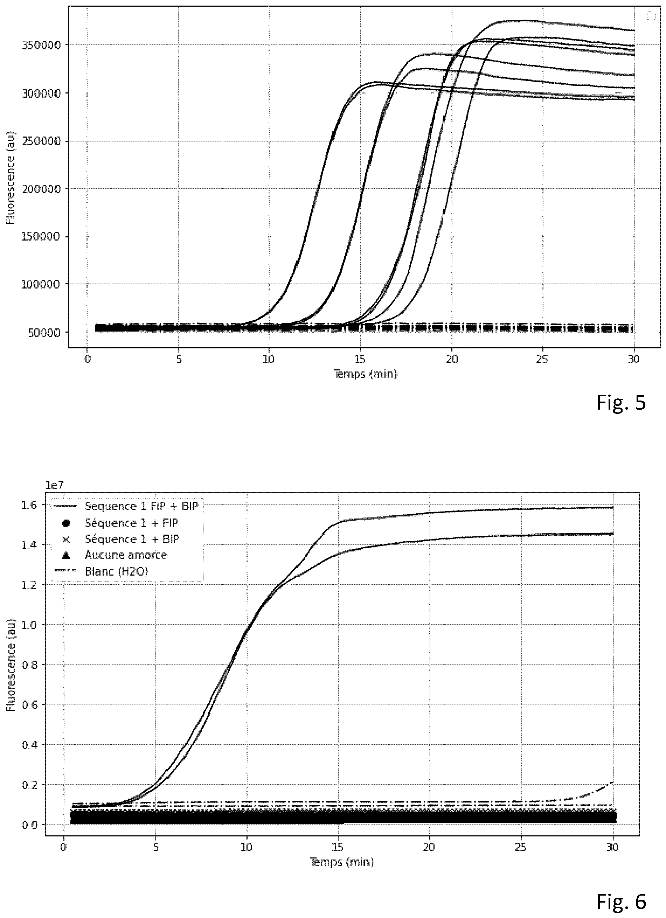

La séquence 1 n'est amplifiée qu'en la présence de ses amorces spécifiques i.e. FIP1 et BIP1 (Figure 4), de même pour la séquence 2 qui n'est amplifiée qu'en présence de ses amorces spécifiques i.e. FIP2 et BIP2 (Figure 5). Cela est une preuve de la sélectivité des amorces vis-à-vis de leur séquence, et donc valide l'utilisation de ces conceptions pour réaliser un contrôle interne en solution qui ne produira pas d'amplification non spécifique vis-à-vis d'autres amorces présentes en solution. Cela prouve la robustesse des séquences proposées pour leur utilisation dans des solutions contenant d'autres analytes ou séquences oligonucléotides.

### 4. Preuve de la spécificité des séquences proposées.

Afin de prouver la spécificité du test, une amplification de la séquence 1 est réalisée avec toutes, une seule ou aucune des amorces associées. Dans le cas où une seule ou aucune amorce n'est présente, aucune amplification n'est observée (Figure 6). Cela valide bien la sélectivité de l'amplification de la séquence conçue vis-à-vis des amorces associées.

### Références

[1] Brevet US 6,410,278 publié le 25 juin 2002.
[2] Thompson & Lei, 2020, « Mini review: Recent progress in RT-LAMP enabled COVID-19 détection », Sensors and Actuators Reports, vol. 2, Art. N° 1.
[3] Abdullah AL-maskri et al, 2020, « Reverse transcription-based loop-mediated isothermal amplification strategy for real-time miRNA détection with phosphorothioated probes », Analytica Chimica Acta, vol. 1126, pages 1-6.
[4] Gines et al, 2019, « Emerging isothermal amplification technologies for microRNA biosensing: Applications to liquid biopsies », Molecular Aspects of Medicine, vol. 72, page 100832.
[5] Aubret et al, 2022, « Development of an Innovative Quantification Assay Based on Aptamer Sandwich and Isothermal Dumbbell Exponential Amplification », Anal. Chem., vol. 94, pages 3376-3385.
[6] Ball et al, 2016, « Quenching of Unincorporated Amplification Signal Reporters in Reverse-Transcription Loop-Mediated Isothermal Amplification Enabling Bright, Single-Step, Closed-Tube, and Multiplexed Détection of RNA Viruses », Anal. Chem., vol. 88, pages 3562-3568.
[7] Tanner et al, 2012, « Simultaneous multiple target détection in real-time loop-mediated isothermal amplification », BioTechniques, vol. 53, pages 81-89.
[8] Quyen et al, 2022, « Multiplex détection of pathogens using solid-phase loop-mediated isothermal amplificationon a supercritical angle fluorescence array for point-of-care applications », ACS Sensors, vol. 7, pages 3343-3351.
[9] Demande de brevet EP 3878971 publiée le 15 septembre 2021.
[10] Demande de brevet EP 22195865.5 déposée le 15 septembre 2022.
[11] Demande de brevet EP 3363913 publiée le 22 août 2018.

## Revendications

1. Procédé pour contrôler la qualité d'une amplification isotherme médiée par des boucles d'un mélange réactionnel contenant un contrôle interne et éventuellement au moins une séquence nucléotidique d'intérêt, ledit procédé comprenant
a) la co-amplification isotherme médiée par des boucles dudit contrôle interne et de ladite séquence nucléotidique d'intérêt éventuellement présente dans ledit mélange réactionnel et
b) le contrôle de la qualité de l'amplification isotherme médiée par des boucles en détectant le produit de l'amplification dudit contrôle interne,
**caractérisé en ce que** ledit contrôle interne se présente sous forme d'un oligonucléotide de formule (I) :
(F1c)ₘ-F2-(F0')ₙ-F1-(Int)ₚ-B1c-(B0')_{q}-B2c-(B1)ᵣ (I)
dans laquelle
m, n, p, q et r, identiques ou différents, sont égaux à 0 ou 1,
la portion F2 comprend de 8 à 30 nucléotides,
la portion F1 comprend de 10 à 35 nucléotides,
Int séparant la portion F1 et la portion B1c est, lorsque p est égal à 0, une liaison covalente et, lorsque p est égal à 1, une portion comprenant au moins un nucléotide,
la portion B1c comprend de 10 à 35 nucléotides,
la portion B2c comprend de 8 à 35 nucléotides,
F1c représente, lorsque m est égal à 0, l'extrémité 5' de la portion F2 et, lorsque m est égal à 1, une portion dont la séquence nucléotidique est complémentaire à la séquence nucléotidique de la portion F1,
F0' représente, lorsque n est égal à 0, une liaison covalente liant les portions F2 et F1 et, lorsque n est égal à 1, une portion comprenant au moins un nucléotide,
B0' représente, lorsque q est égal à 0, une liaison covalente liant les portions B1c et B2c et, lorsque q est égal à 1, une portion comprenant au moins un nucléotide, et
B1 représente, lorsque r est égal à 0, l'extrémité 3' de la portion B2c et, lorsque r est égal à 1, une portion dont la séquence nucléotidique est complémentaire à la séquence nucléotidique de la portion B1c et
**en ce que** ledit mélange réactionnel contient au plus deux amorces utiles pour l'amplification isotherme médiée par des boucles dudit contrôle interne et éventuellement une autre séquence nucléotidique pour la détection de l'amplification dudit contrôle interne.

2. Procédé de contrôle selon la revendication 1, **caractérisé en ce que** ledit oligonucléotide répond à la formule (II) ci-après :
5'-F2-F1-(Int)ₚ-B1c-B2c-3' (II)
avec p et les portions F2, F1, Int, B1c et B2c tels que définis à la revendication 1.

3. Procédé de contrôle selon la revendication 1, **caractérisé en ce que** ledit oligonucléotide répond à la formule (III) ci-après :
5'-F1c-F2-F0'-F1-(Int)ₚ-B1c-B0'-B2c-B1-3' (III)
avec p et les portions F1c, F2, F0', F1, Int, B1c, B0', B2c et B1 tels que définis à la revendication 1.

4. Procédé de contrôle selon la revendication 1, **caractérisé en ce que** ledit oligonucléotide répond à la formule (IV) ci-après :
5'-F2-F0'-F1-(Int)ₚ-B1c-B0'-B2c-B1-3' (IV)
avec p et les portions F2, F0', F1, Int, B1c, B0', B2c et B1 tels que définis à la revendication 1.

5. Procédé de contrôle selon la revendication 1, **caractérisé en ce que** ledit oligonucléotide répond à la formule (V) ci-après :
5'-F1c-F2-F0'-F1-(Int)ₚ-B1c-B0'-B2c-3' (V)
avec p et les portions F1c, F2, F0', F1, Int, B1c, B0' et B2c tels que définis à la revendication 1.

6. Procédé de contrôle selon l'une quelconque des revendications 1 à 5, **caractérisé en ce que** ledit oligonucléotide se trouve en solution dans le mélange réactionnel.

7. Procédé de contrôle selon l'une quelconque des revendications 1 à 5, **caractérisé en ce que** ledit oligonucléotide est immobilisé au niveau de la surface d'un support solide avec lequel le mélange réactionnel est en contact.

8. Procédé de contrôle selon la revendication 7, **caractérisé en ce que** cette immobilisation est indirecte et fait intervenir, en tant que bras espaceur, une séquence nucléotidique complémentaire à une partie de la séquence nucléotidique dudit oligonucléotide et fixée à la surface du support.

9. Procédé de contrôle selon l'une quelconque des revendications 1 à 8, **caractérisé en ce que** ledit mélange réactionnel comprend, comme seules amorces impliquées dans l'amplification et la détection de l'amplification dudit oligonucléotide, les amorces FIP et BIP.

10. Procédé de contrôle selon l'une quelconque des revendications 1 à 8, **caractérisé en ce que** ledit mélange réactionnel comprend, comme seules amorces impliquées dans l'amplification et la détection de l'amplification dudit oligonucléotide, les amorces FIP et BIP, l'une des deux étant marquée, à son extrémité 5' ou 3', par un premier fluorochrome, et une troisième séquence nucléotidique marquée par un second fluorochrome.

11. Procédé de contrôle selon l'une quelconque des revendications 1 à 10, **caractérisé en ce que** la détection des produits d'amplification dudit oligonucléotide se fait :
- par mesure de la fluorescence,
- en détectant les sous-produits de la réaction d'amplification comme, par exemple, des cristaux de pyrophosphate, ou
- par mesure de la variation de pH.

12. Kit pour contrôler la qualité d'une amplification LAMP consistant en un oligonucléotide tel que défini à l'une quelconque des revendications 1 à 5 et les amorces FIP et BIP.

13. Kit pour contrôler la qualité d'une amplification LAMP consistant en un oligonucléotide tel que défini à l'une quelconque des revendications 1 à 5, les amorces FIP et BIP, l'une des deux étant marquée, à son extrémité 5' ou 3', par un premier fluorochrome, et une troisième séquence nucléotidique marquée par un second fluorochrome.
